# EUROPEAN PATENT SPECIFICATION

(11) **EP 1 956 987 B1**
(45) Date of publication and mention of the grant of the patent: **12.01.2011**
(21) Application number: 06778048.6
(22) Date of filing: 28.07.2006
(51) Int. Cl.: A61B 17/064, A61B 17/88, A61B 17/82

(54) **STERNUM SUTURING STAPLE AND APPARATUS ADAPTED TO POSITION AND TIGHTEN THE SAME**
STERNUM-NAHTKLAMMER UND GERÄT ZUM POSITIONIEREN UND ANZIEHEN DER KLAMMER
AGRAFE DE SUTURE STERNALE ET SON APPAREIL DE MISE EN PLACE ET DE SERRAGE

(30) Priority: 28.07.2005 IT MI20051461
(43) Date of publication of application: 20.08.2008
(73) Proprietor: Puricelli, Cesare, 33034 Fagagna (IT); Ponte, Gianni, 33100 Udine (IT)
(72) Inventor: Puricelli, Cesare, 33034 Fagagna (IT); Ponte, Gianni, 33100 Udine (IT)
(74) Representative: Gervasi, Gemma
(86) International application number: PCT/EP2006/064778
(87) International publication number: WO 2007/014910

(56) References cited:
- WO-A2-2004/016181
- US-A- 4 723 540
- US-A- 6 051 007
- US-A1- 2002 032 465

## Description

### Field of the invention

The present invention relates to a sternum suturing staple; it is specifically employed after surgery, in which sternotomy was required, as in heart surgery. There is also described an apparatus adapted to position and tighten said staple.

### Prior art

At present, stemotomy represents the most commonly employed access system to the mediastinum in surgery such as heart surgery. Upon ending the operation, the closure of the sternum is commonly performed by passing metal wires around the two halves, in which it has been divided; the wires are wound in front of the sternum in order to tighten them, thus closing the two parts of the bone together. Each wire may be passed once or twice, in this latter case it is crossed. Six wires passed once, or three twice are commonly employed for the complete suture of the sternum. The technique involves several problems. As the closure is manually carried out by the surgeon, the wire tension is generally uneven, causing reduced efficiency of the closure and possible dehiscence (the tighter lace will loosen as it tends to saw through the bone). Moreover, there often occurs a certain displacement of the two drawn-together halves of the bone.

Moreover, the force required to pass the wires is considerable and a sharp needle is needed with the risk of the surgeon injuring himself. As far as the patient is concerned, the passages through and around the sternum involve the risk of injuries to the inner thoracic arteries and also to the pleuras and lungs, and the result greatly depends on the ability of the surgeon. There may also arise the subsequent occurrence of pain, the dehiscence of the wound, surface and deep infections, the latter (mediastinitises) being potentially lethal.

There are also used small, bands provided with a locking system (a serrated end engaged with an appropriate closure system on the other end, so as to allow the surgeon to tighten the small band and maintain it tightened after it is released). However, this system exhibits many of the problems connected to the use of metal wire.

Moreover, because of biocompatibility problems, and due to the need of using a non-magnetic material, thus avoiding problems upon a possible subsequent use on the patent of diagnostic apparatuses, such as nuclear magnetic resonance, whose use is increasingly widespread, it is required that these surgical devices, intended to permanently remain within the body of the patient; are made of titanium, a notoriously poorly flexible material, which increases the difficulties in positioning and application during surgery.

There have been suggested staples made of materials known as "shape memory alloys" to be placed over the sternum to clamp the two halves. These are alloys capable of expanding at a low temperature (thus allowing positioning the staple over the sternum) and restoring the original shape at the body temperature. They are very expensive and, moreover, the staple tension may not be adjusted, therefore not solving the problems regarding uneven tension in the various staples, thus creating considerable problems due to different shapes and sizes that the sternum may have in different patients. Moreover, there is a stand-by time for the heating and therefore for the closure of the staple,

Other types of suggested staples envisage the puncturing of the sternum by means of punch pliers, but it is not desirable to weaken the sternum structure by this practice, besides the fact that the preparation of the sternum requires precision, further complicating the operation.

WO 2004/016181 discloses a sternum suturing staple having a central cylindrical hollow body with right and left threaded ends in which two teeth are screwed. In order to draw the teeth together it is necessary to rotate the whole central body by means of an apparatus provided with friction. This kind of staple presents several drawbacks. First of all, it is not possible to apply high values of torque to the central body by friction; secondly, it is not possible to easily cut the central body in case it is necessary to quickly remove the staple without unscrewing the staple; furthermore, the overall dimensions of the central body itself and of the apparatus are not compatible with the free space which exists around the sternum.

### Summary

The problems discussed above have now been solved according to the present invention by means of a new type of sternum suturing staple, comprising a central body having at least two threaded portions with opposite threads and at least two side members each having a threaded part adapted to reciprocally couple to one of the threaded parts of the central body and a jaw adapted to be positioned laterally to a sternum, and in which the jaws are adapted to cooperatively clamp the sternum so as to suture it.

Preferably, said jaw exhibits at least a first tooth in intermediate position adapted to engage with the sternum.

Preferably, said jaw exhibits a second tooth, adapted to engage the sternum (simultaneously or as an alternative to the first), positioned at one end of the jaw opposite to the threaded part.

The jaw preferably exhibits a concave part facing the sternum position, i.e. on the side of the central body.

### List of the figures

The present invention will now be illustrated by means of the detailed description of a preferred, although non-limiting, embodiment provided merely by way of example, with the aid of the attached figures, wherein:
Figures 1a and 1b schematically represent a front section view of a staple according to the present invention, to different screwing degrees of the threaded parts;
Figure 2 schematically represents a front view of a staple according to the present invention having a different size of the jaws;
Figure 3 schematically represents the central body of a staple according to the present invention;
Figure 4 schematically represents, in partial section, the front view of a side member of a staple according to the present invention;
Figure 5 schematically represents the side view of a side member of a staple according to the present invention;
Figure 6a schematically represents a perspective view of an apparatus to position and screw staples according to the present invention;
Figure 6b schematically represents a perspective view of the apparatus of Figure 6a having a staple positioned and held by the same.

### Detailed description

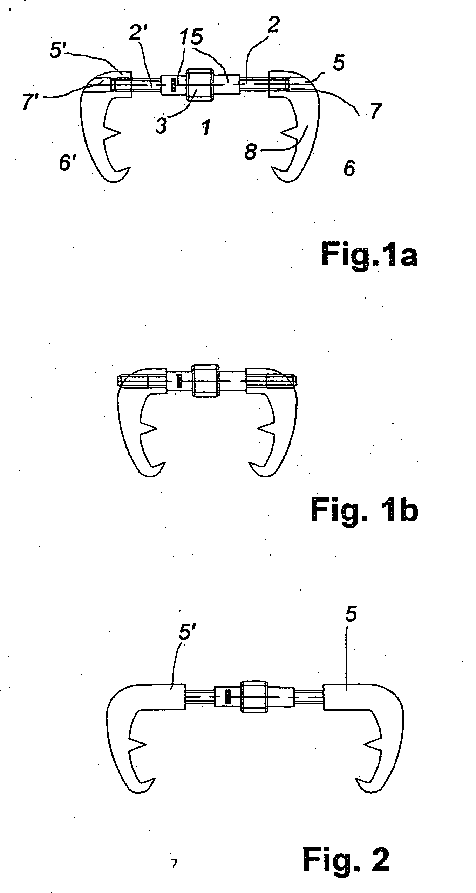

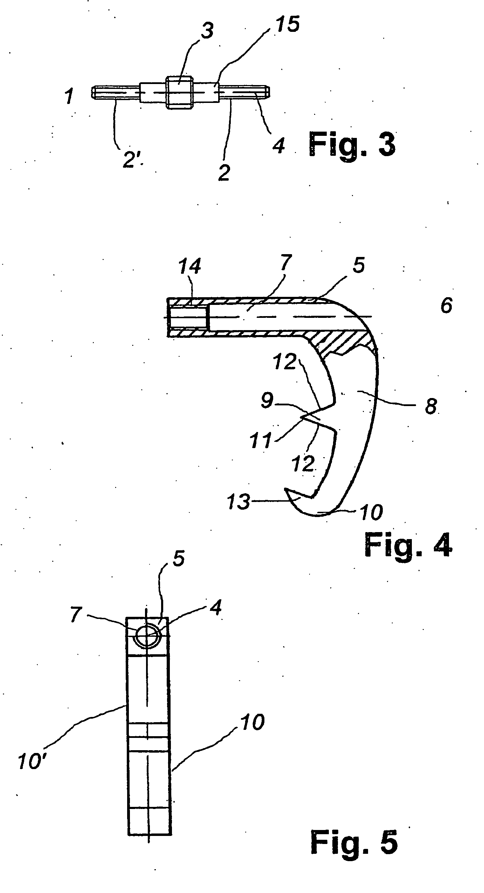

A staple according to the present invention is now described with reference to Figures 1a, 1b, 2 and 3. A central body 1 exhibits threaded portions 2 and 2', one having right-headed thread, the other halving left-headed thread. According to a preferred embodiment of the invention, the body is essentially cylindrical (specifically see Figure 3) and the threads of portions 2 and 2' are outer threads. Preferably, there exists a crown 3, which may be knurled, or it may preferably exhibit a gear toothing to facilitate the manual grip, or preferably the grip by an appropriate apparatus.

The threaded portions are adapted to engage with the corresponding threaded parts 5 and 5' of side members 6 and 6'. Such portions exhibit, according to a preferred embodiment of the invention, internally threaded holes 7 and 7', adapted to receive the threaded portions of the central body. According to a preferred embodiment of the invention, these are through-holes and may be threaded along only part 14 of their length, for example from 2 to 4 mm, while the remainder of the hole may accommodate the threaded portion of the central body without engagement, having a larger diameter than the external diameter of the threaded portion. Such part of the length may be chosen on the basis of requirements for the mechanical resistance of the staple and for overall screwing resistance.

By rotating the central body about its longitudinal axis 4, a symmetrical motion of the side members is determined, which will be drawn together and apart along the direction of axis 4, depending on the rotation direction of the central body, by means of the opposite threads.

Comparing Figures 1a and 1b, it may be noted how the closure of the staple may be adjusted by operating on the central body.

A side member is shown in more detail with reference to Figure 5.

Similarly to the opposite (and specular) side member 6' of Figure 1a, side member 6 exhibits a jaw 8 adapted to be positioned laterally to the sternum in an intercostal space. The sternum may thus be positioned between the two jaws of the two side members of the staple. Drawing the side members together, by operating on the central body, the two halves of a sternum that has been subjected to median sternotomy, may be drawn and clamped together, thus carrying out the suture. Generally, several staples will be needed, in a variable number according to the size and conditions of the sternum, for example from four to six.

Jaw 8 exhibits a first tooth 9 facing the position occupied by the sternum, substantially facing the jaw of the opposite side member.

Figure 5 shows that the side member may be substantially delimited between two reciprocally parallel planes 10 and 10', also parallel to axis 4 of the threaded holes and therefore of central body, when mounted. Jaw 8 may have a curved shape, with a concavity facing the opposite jaw, i.e. facing the part intended to be facing the sternum. The first tooth 9 may have different shapes and be located in an intermediate position between the threaded part and the free end 10 of the jaw. For example, it may have triangular shape, with preferably acute vertex 11, for example, from 40 to 50°.

The tooth is adapted to engage with the sternum in different ways, for example, laterally inserting into it or, in some way, locking it against one of the lateral profiles 12. According to a preferred embodiment of the invention, the jaw exhibits, at the free end, a second tooth 13 also substantially directed to the same side of the first tooth; it may also be partly directed to the central body, as shown. The second tooth will also be appropriate to engage the sternum as the first tooth. In accordance with the thickness of the sternum, the first tooth only, or both teeth, may be engaged with the sternum. The first tooth may advantageously be essentially located halfway along the jaw, i.e. the distance of vertex 11 from axis 4 of hole 7 and that of the vertex from the tangent, parallel to said axis, to the free end 10 may have a ratio in the range from 1:2 to 2:1; and may preferably be substantially the same. The distance of said tangent from said axis may, for example, be from 15 to 20 mm. With such dimensions, the staple may be easily adapted to all of the normally occurring sizes of the human sternum, different engagement modes being possible and also thanks to the concavity facing the sternum. Moreover, the shape of end 10 avoids possible injuries to the underlying organs (for example the pericardium), also thanks to the intercostal position of the jaw; this is unlike systems in which the staple is inserted in holes obtained in the sternum, systems leading to the need for the staple to have different sizes, given the proximity of the underlying organs, which at certain positions may be nearly in contact with the sternum. The height of the teeth may, for example, be in the range from 0,5 to 3 mm. As the jaw is intended to be inserted in an intercostal space from free end 10, end 10 is preferably rounded on the side opposite to the threaded part of the side member, so as not to be detrimental.

As shown in Figures 1 a and 1b, the arrangement with through-holes into the side members allows a considerable adaptability of the staple, even with a single size of the central body having overall length that may be, for example, from 20 to 35 mm. It was found advantageous to have different size staples, differing from one another only in the total length of the threaded parts (compare with Figures 1a and 2), in order to adjust to the various widths of the sternum. This makes the manufacture of staples simple and cost-effective. The other dimensions may anyway be varied, if desired.

The different lengths of the threaded parts (in any case also comprising the whole of the non-threaded hole, extending from the jaw to the central body engaging end) has the advantage of reducing the length of the threaded portion of the central body protruding from the side member, once the staple has been positioned on the sternum, in order to reduce possible flexing stresses on the central body that will be appropriately sized anyway. Between the threaded portion and the central body crown (Figure 3) there preferably exists a non-threaded segment 15, having a diameter that may be equivalent or longer than the external diameter of the threaded portion. This provides a central body part that is not engaged with the side member, which may be easily cut by means of cutters or other appropriate tool. A further advantage of the present invention, particularly if the outer thread is on the central body as in the example, is the possibility of carrying out an emergency reopening of the sternum by cutting the staples likewise, which may then be removed, without proceeding to the total removal (by unscrewing), that would involve times which may become critical in case of emergency. Appropriate dimensions of the outer diameter of the threaded portion may vary, for example, from 1 to 3 mm, for example 2 mm, with metric screw thread being right-headed or left-headed depending on the case.

Crown 3 is adapted to be rotated about axis 4 of the central body. Preferably, it is adapted to be driven by a member (for example a roller, preferably a toothed roller) of a suitable apparatus provided with a dynamometer device for adjusting the tightening torque, and therefore the tension to be applied to the staple once positioned mounting the sternum.

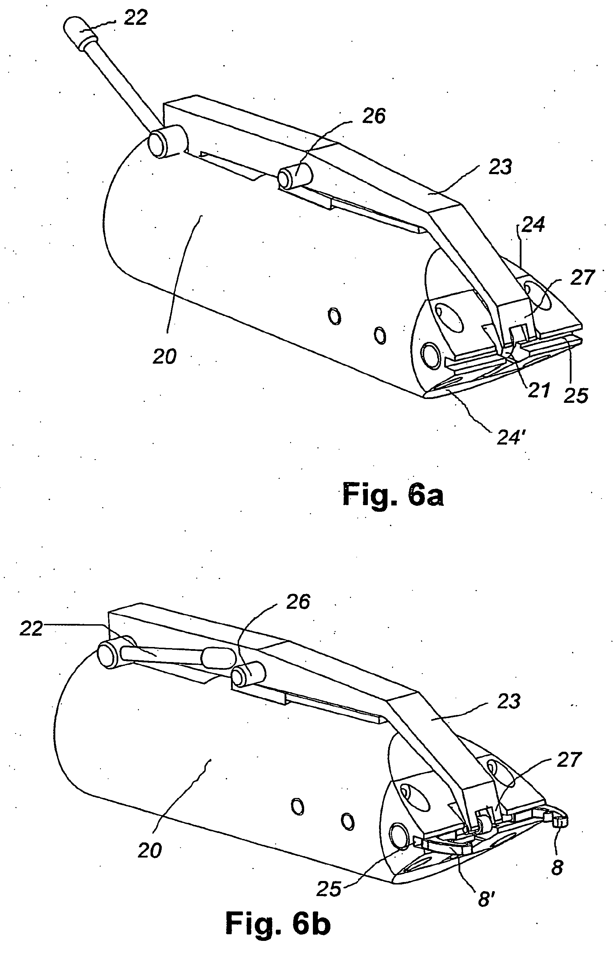

Tension may be applied to a staple by means of an apparatus such as that shown in Figures 6a and 6b, also adapted to operate the correct positioning thereof. It exhibits a motor, appropriately accommodated in a casing connectable to element 20, for example inserted in element 20, which may exhibit an appropriate seat adapted to accommodate the casing, and driven, for example, by batteries or rechargeable batteries. The casing may be extractible and may have means adapted to engage with the motion transmission means of element 20. The motor may also be otherwise accommodated in element 20. Element 20 or the casing may exhibit controls to appropriately drive the motor. By motion transmission means (wheels and gears) that may comprise roller, or preferably, gear 21, the apparatus will rotate the crown of the central body (which may be a toothed wheel corresponding to the gear or a knurling wheel, in accordance with the structure of roller 21) of a staple held in position by suitable holding means.

The apparatus is preferably provided with a mechanical or electrical clutch or with any other type of dynamometer device, preferably presettable, in order to apply a predetermined tension to the staple. In the shown case, the holding means is a tongue 23 that may be removed by operating lever 22 after screwing has been carried out. Tongue 23 may move from an open position (Figure 6a) to a closed position (Figure 6b). It is adapted to hold the staple with the central body and the threaded parts of the staple in the adapted groove 25, as in Figure 6b. The roller or gear 21 appropriately protrudes from element 20 at the central part of the groove so as to engage with the staple crown when the latter is held. The staple may be inserted in the groove and then locked by the tongue. Jaws 8 and 8' may be inserted in the opposite intercostal spaces and may then be drawn together by means of the operation of the motor until the two sternum halves are sutured with the desired tension determined by the dynamometer device. The staple may be released by operating on lever 22. Element 20, which may be cylindrical, preferably exhibits two surfaces 24 and 24' arranged as a V, with groove 25 in the vertex, so as to simplify the positioning on the patient without unnecessary obstruction. The groove has a section corresponding to the cross section of the threaded parts of the side members of the staple, so as to prevent it from rotating during screwing, but such that it allows the side members to be slided. Preferably, the cross section (Figure 5) is rectangular or square, with a side longer than the diameter of hole 7, for example from 2 to 5 mm. Lever 22, for example rotatably attached to element 20, may operate on a cam positioned internally to an end of tongue 23, which may be hinged on pin 26, appropriately attached to element 20, so that the oscillation determines the motion of the other end 27 in open and closed positions. The lever is easily handled even during the positioning on the patient, as it is preferably on the opposite side of element 20 with respect to the groove. End 27 may be fork-shaped so as to take a position mounting the crown of the staple, not interfering with it, but holding the staple, for example in segments 15.

Other embodiments of the staple holding system may be envisaged. The staple is intended for the suturing of a (human or even animal) sternum, specifically after stemotomy. The intercostal spaces, in which the staple jaws are to be inserted, may be surgically prepared according to known techniques, similar for example to those used for the insertion of other types of suturing systems.

The staple may be made of any suitable material, preferably biocompatible as the sternal suture is generally intended to be permanently left inside the patient. According to a preferred embodiment of the invention, it is integrally made of titanium.

The invention also comprises an apparatus for positioning and tightening staples as described above. The apparatus may also be suitable for disassembling the staples by unscrewing. A system for inverting the rotation direction of the central body may be envisaged if desired, or the direction in which the staple is inserted may simply be changed.

## Claims

1. A sternum suturing staple, comprising a central body (1) having at least two threaded portions (2, 2') with opposite threads and at least two side members (6, 6') each having a threaded part (5, 5') adapted to reciprocally couple with one of the threaded parts of the central body and a jaw (8, 8') adapted to be positioned laterally to a sternum, and wherein the jaws are adapted to cooperatively clamp the sternum so as to suture it, **characterized in that** said threaded portions (2, 2') of the central body exhibit outer threads and **in that** said central body (1) exhibits a crown (3) adapted to be rotated manually or by means of a suitable apparatus about a longitudinal axis (4) of the central body, a non-threaded segment (15) being provided on said central body (1) between said crown (3) and said threaded portions (2, 2')."

2. A staple according to claim 1, wherein said threaded parts of the side members (2, 2') exhibit through-holes (7, 7'), internally threaded for a determined length (14).

3. A staple according to claim 1 or 2, wherein said jaw exhibits at least a first tooth (9) in intermediate position adapted to engage with the sternum.

4. A staple according to any of the previous claims, wherein said jaw exhibits a second tooth (13), adapted to engage with the sternum, positioned at one end of the jaw opposite to the threaded part.

5. A staple according to any of the previous claims, wherein said jaw exhibits a concave part facing the central body.

6. A staple according to any of the previous claims, wherein said jaw exhibits a rounded free end.

7. A staple according to claim 3, wherein said first tooth has a triangular shape with vertex (11) in the range from 40 to 50°.

8. A staple according to any of she previous claims, wherein said crown (3) is toothed and gear-shaped.

9. A staple according to any of the previous claims, made of biocompatible material.

10. A staple according any claims 1 to 8 made of titanium.

11. A staple according to any of the previous claims, wherein said threaded parts of the side members have square or rectangular cross section.

12. An apparatus adapted to position and tighten a staple according to any of the previous claims, comprising a motor and having a element (20) exhibiting a groove (25) adapted to accommodate and hold the central body and the threaded parts of the side members and means (21) adapted to rotate said central body about its longitudinal axis (4).

13. An apparatus according to claim 12.adapted to position and tighten a staple according to claim 8, wherein said means comprises a gear (21) adapted to engage with said crown (3).

14. An apparatus according to claim 12 or 13, comprising a dynamometer system to control the tightening torque.

## Patentansprüche

1. Sternum-Nahtklammer, umfassend einen Hauptkörper (1), der wenigstens zwei Gewindeabschnitte (2, 2') mit entgegengesetzten Gewinden und wenigstens zwei Seitenbauteile (6, 6'), die jeweils einen Gewindeteil (5, 5') aufweisen, der geeignet ist, um sich mit einem der Gewindeteile des Hauptkörpers reziprok zu koppeln, und eine Backe (8, 8') umfasst, die geeignet ist, um seitlich an einem Sternum positioniert zu sein, und wobei die Backen das Sternum zusammenwirkend klemmen können, um es so zu nähen, **dadurch gekennzeichnet, dass** die Gewindeabschnitte (2, 2') des Hauptkörpers Außengewinde aufweisen, und dass der Hauptkörper (1) eine Kalotte (3) aufweist, die manuell oder mittels einer geeigneten Vorrichtung um die Längsachse (4) des Hauptkörpers gedreht werden kann, wobei ein Segment (15) ohne Gewinde an dem Hauptkörper (1) zwischen der Kalotte (3) und den Gewindeabschnitten (2, 2') vorgesehen ist.

2. Klammer nach Anspruch 1, wobei die Gewindeteile der Seitenbauteile (2, 2') Durchgangslöcher (7, 7') aufweisen, die für eine bestimmte Länge (14) ein Innengewinde haben.

3. Klammer nach Anspruch 1 oder 2, wobei die Backe wenigstens einen ersten Zahn (9) in der Zwischenposition aufweist, der in das Sternum eingreifen kann.

4. Klammer nach einem der vorhergehenden Ansprüche, wobei die Klammer einen zweiten Zahn (13) aufweist, der in das Sternum eingreifen kann, der an einem Ende des Backen gegenüber dem Gewindeteil positioniert ist.

5. Klammer nach einem der vorhergehenden Ansprüche, wobei die Backe ein konkaves Teil aufweist, das dem Hauptkörper gegenüberliegt.

6. Klammer nach einem der vorhergehenden Ansprüche, wobei die Backe ein abgerundetes freies Ende aufweist.

7. Klammer nach Anspruch 3, wobei der erste Zahn eine dreieckige Form mit einer Spitze (11) im Bereich von 40 bis 50° aufweist.

8. Klammer nach einem der vorhergehenden Ansprüche, wobei die Kalotte (3) gezahnt und Ritzel förmig ist.

9. Klammer nach einem der vorhergehenden Ansprüche, die aus Biomaterial hergestellt ist.

10. Klammer nach einem der Ansprüche 1-8, die aus Titan hergestellt ist.

11. Klammer nach einem der vorhergehenden Ansprüche, wobei die Gewindeteile der Seitenbauteile einen quadratischen oder rechtwinkligen Querschnitt aufweisen.

12. Vorrichtung, die geeignet ist, um eine Klammer gemäß den vorhergehenden Ansprüchen zu positionieren und anzuziehen, die einen Motor umfasst und die ein Element (20), das eine Nut (25), die den Hauptkörper und die Gewindeteile der Seitenbauteile aufnehmen und halten kann, und Mittel (21) aufweist, die den Hauptkörper um seine Längsachse (4) drehen können.

13. Vorrichtung nach Anspruch 12, die eine Klammer gemäß Anspruch 8 positionieren und anziehen kann, wobei die Mittel ein Getriebe (21) umfassen, das in die Kalotte (3) eingreifen kann.

14. Vorrichtung nach Anspruch 12 oder 13, umfassend ein Dynamometer-System, um das Anzieh-Drehmoment zu steuern.

## Revendications

1. Agrafe de suture sternale, comprenant un corps central (1) ayant au moins deux parties filetées (2, 2') avec des filets opposés et au moins deux éléments latéraux (6, 6'), ayant chacun une partie filetée (5, 5'), adaptée pour se raccorder réciproquement à une des parties filetées du corps central et une mâchoire (8, 8') adaptée pour être positionnée latéralement par rapport au sternum, et dans laquelle les mâchoires sont adaptées pour se fixer de manière coopérative à un sternum de façon à le suturer,
**caractérisée en ce que** lesdites parties filetées (2, 2') du corps central présentent des filets extérieurs et **en ce que** ledit corps central (1) présente une couronne (3) adaptée pour être mise en rotation manuellement ou au moyen d'un appareil adapté autour d'un axe longitudinal (4) du corps central, un segment non-fileté (15) étant ménagé sur ledit corps central (1) entre ladite couronne (3) et lesdites parties filetées (2, 2').

2. Agrafe selon la revendication 1, dans laquelle lesdites parties filetées des éléments latéraux (2, 2') présentent des orifices passants (7, 7') intérieurement filetés sur une longueur déterminée (14).

3. Agrafe selon la revendication 1 ou 2, dans laquelle ladite mâchoire présente au moins une première dent (9) en position intermédiaire adaptée pour se mettre en prise avec le sternum.

4. Agrafe selon l'une quelconque des revendications précédentes, dans laquelle ladite mâchoire présente une seconde dent (13), adaptée pour se mettre en prise avec le sternum, placée à une extrémité de la mâchoire, opposée à la partie filetée.

5. Agrafe selon l'une quelconque des revendications précédentes, dans laquelle ladite mâchoire présente une partie concave orientée vers le corps central.

6. Agrafe selon l'une quelconque des revendications précédentes, dans laquelle ladite mâchoire présente une extrémité libre arrondie.

7. Agrafe selon la revendication 3, dans laquelle ladite première dent a une forme triangulaire, avec un sommet (11) dans la gamme de 40 à 50°.

8. Agrafe selon l'une quelconque des revendications précédentes, dans laquelle ladite couronne (3) est dentée et en forme d'engrenage.

9. Agrafe selon l'une quelconque des revendications précédentes, faite d'un matériau biocompatible.

10. Agrafe selon l'une quelconque des revendications 1-8, faite de titane.

11. Agrafe selon l'une quelconque des revendications précédentes, dans laquelle lesdites parties filetées des éléments latéraux ont une section transversale carrée ou rectangulaire.

12. Appareil adapté pour positionner et serrer une agrafe selon l'une quelconque des revendications précédentes, comprenant un moteur et ayant un élément (20) présentant une cannelure (25) adaptée pour recevoir et maintenir le corps central et les parties filetées des éléments latéraux et des moyens (21) adaptés pour faire tourner ledit corps central autour de son axe longitudinal (4).

13. Appareil selon la revendication 12, adapté pour positionner et serrer une agrafe selon la revendication 8, dans lequel lesdits moyens comprennent un engrenage (21) adapté pour mettre en prise ladite couronne (3).

14. Appareil selon la revendication 12 ou 13, comprenant un système dynamomètre pour contrôler le couple de serrage.
